# EUROPÄISCHE PATENTSCHRIFT

(11) **EP 3 048 881 B1**
(45) Veröffentlichungstag und Bekanntmachung des Hinweises auf die Patenterteilung: **18.07.2018**
(21) Anmeldenummer: 14781107.9
(22) Anmeldetag: 25.09.2014
(51) Int. Cl.: A01M 1/02, G01N 29/14, G01N 29/44

(54) **VORRICHTUNG UND VERFAHREN ZUM ERHALTEN EINER INFORMATION ÜBER EIN ODER MEHRERE LEBEWESEN**
DEVICE AND METHOD FOR OBTAINING INFORMATION ABOUT ONE OR MORE INVERTEBRATES
DISPOSITIF ET PROCÉDÉ POUR OBTENIR UNE INFORMATION SUR UN OU PLUSIEURS ORGANISMES

(30) Priorität: 26.09.2013 DE 102013219474
(43) Veröffentlichungstag der Anmeldung: 03.08.2016
(73) Patentinhaber: Fraunhofer-Gesellschaft zur Förderung der angewandten Forschung e.V., 80686 München (DE)
(72) Erfinder: RÖDER, Olaf, 01326 Dresden (DE); SCHUBERT, Lars, 01920 Schönteichen OT Petershain (DE); TSCHÖPE, Constanze, 01558 Großenhain (DE); FRANKENSTEIN, Bernd, 01454 Ullersdorf (DE); LIESKE, Uwe, 01109 Dresden (DE)
(74) Vertreter: Burger, Markus
(86) Internationale Anmeldenummer: PCT/EP2014/070575
(87) Internationale Veröffentlichungsnummer: WO 2015/044316

(56) Entgegenhaltungen:
- FR-A1- 2 825 801
- US-A- 4 671 114
- US-A- 4 991 439
- US-A- 5 473 942

## Beschreibung

Ausführungsbeispiele der vorliegenden Erfindung beziehen sich auf eine Vorrichtung und ein Verfahren zum Erhalten einer Information über ein oder mehrere Lebewesen. Weitere Ausführungsbeispiele beziehen sich auf ein Verfahren und eine Vorrichtung zur selektiven Anregung, Detektion und Überwachung von Tierpopulationen, wie z.B. Insekten, sowie zur Bestimmung spezifischer Entwicklungs- und Verhaltenszustände.

Ein Bereich, in dem eine Detektion und/oder Überwachung von Tierpopulationen erforderlich ist, ist die Schädlingsüberwachung und Schädlingsbekämpfung.

Mehr als 10% der weltweiten Getreideernte wird durch tierische Schädlinge vernichtet. Diese Verluste betragen weltweit ca. 200 Millionen Tonnen Nahrungs- und Futtermittel pro Jahr. Der daraus entstehende volkswirtschaftliche Schaden beläuft sich weltweit auf etwa 20 Milliarden Euro pro Jahr. Allein in Deutschland werden jährlich ca. 40 Millionen Tonnen Getreide gelagert. Die Lebensmittelverordnung schreibt dabei Mühlen und Verarbeitern vor, keine Produkte anzunehmen bzw. zu verarbeiten, die tierische Schädlinge enthalten. Beispielsweise ist der gefährlichste Vorrats-Schädling in Europa der Kornkäfer.

Es existieren diverse Verfahren zur Erkennung tierischer Schädlinge in Schüttgut, wie z.B. Getreide. Bei einem bekannten Verfahren werden empfindliche Stabmikrofone in die Schüttung gesteckt und bei Schädlingsbefall entsprechende akustische Signale, wie z.B. Knabbergeräusche, empfangen. Die Knabbergeräusche der Insekten, insbesondere der Insektenlarven, können dabei manuell oder (halb)automatisiert erkannt und bewertet werden.

Mit den bekannten Verfahren zur Erkennung tierischer Schädlinge können die Schädlinge in ihren verschiedenen Entwicklungsstadien jedoch nur dann erkannt werden, wenn diese zum Zeitpunkt der Messung Geräusche verursachen bzw. emittieren. Die Aktivitäten der Schädlinge sind jedoch stark abhängig von der Umgebungstemperatur. Beispielsweise ist bei einer verringerten Temperatur eine Erkennung der Schädlinge nicht oder nur schwer möglich.

Ferner ist zu berücksichtigen, dass unterschiedliche Schädlinge unterschiedliche Geräusche verursachen. Mit den bekannten Verfahren zur Erkennung tierischer Schädlinge lassen sich diese Geräusche praktisch nicht differenzieren, d.h. die Art der Schädlinge kann mit den bekannten Verfahren nicht ermittelt werden.

Darüber hinaus ist zu berücksichtigen, dass Schädlinge in der Regel mehrere Entwicklungsstadien durchleben (z.B. durchlebt der Kornkäfer vier Entwicklungsstadien) und dementsprechend je nach Entwicklungsstadium in Amplitude und Frequenz verschiedene Geräusche emittieren. Mit den bekannten Verfahren zur Erkennung tierischer Schädlinge lassen sich diese Geräusche praktisch nicht in verschiedene Entwicklungsstadien differenzieren.

Des Weiteren ist es mit derzeitigen Verfahren nicht möglich, ein Ausmaß eines Schädlingsbefalls zu erkennen und einen Ort eines beginnenden Schädlingsbefalls, z.B. in einem Silo, zu bestimmen.

Zusammenfassend ist es daher mit den bekannten Verfahren zur Erkennung tierischer Schädlinge nicht möglich, durch eine permanente Überwachung von Vorräten eine nach Schädlingsart, Entwicklungsstadium, Befallsumfang und Siedlungsort definiert angepasste in Umfang und Kosten adäquate Schädlingsbekämpfung frühzeitig einzuleiten und gezielt durchzuführen.

Ein weiterer Bereich, in dem eine Detektion und/oder Überwachung von Tierpopulationen erforderlich ist, ist die Überwachung von Nutztieren, wie z.B. die Überwachung von Bienen in der Bienenhaltung.

Der direkte Nutzen von durch Imker gehaltenen Honigbienen besteht in der Produktion von Honig, Wachs, Propolis und Pollen. Die weltweite Produktion von Honig beträgt ca. 1,5 Mio. Tonnen pro Jahr. Weltweit werden ca. 80 Mio. Bienenvölker von Imkern gehalten. Der indirekte Nutzen der Honigbienen besteht in der Übertragung des Blütenstaubs durch die Nektar- und Pollensammeltätigkeit und ist um ein Vielfaches höher als ihre direkte Leistungen durch Honigproduktion. Der wirtschaftliche Nutzen der Bestäubung pro Jahr wird weltweit auf über 100 Milliarden US-Dollar geschätzt. Die weltweit größten Honigproduzenten mit den meisten Bienenvölkern sind (in dieser Reihenfolge) China, USA, Argentinien, Mexiko, Kanada und Brasilien. In Deutschland wird von ca. 100.000 Imkern mit etwa einer Million Bienenvölkern ein Viertel des jährlichen deutschen Honigverbrauchs erzeugt. Der übrige Anteil wird importiert. Große Imkereien in Europa halten oft 300 oder mehr Bienenvölker, in den USA sind es oft 1000 oder mehr Bienenvölker.
Für den Imker besteht ein enormer Aufwand in der Überwachung und tiergerechten imkerlichen Führung der Bienenvölker, der mit zunehmender Anzahl an Völkern schwer beherrschbar oder gar unmöglich wird. Die Bienenhaltung wird durch Parasiten wie der Varroamilbe, durch hoch-ansteckende Krankheiten, wie z.B. der Amerikanischen Faulbrut, und durch den massiven Einsatz von Pestiziden in der Landwirtschaft erheblich erschwert. Imker benötigen daher idealerweise zu jeder Zeit Informationen über jedes ihrer Völker bezüglich spezifischer Zustände, wie z.B. dem mentalen Zustand, dem Schwarmverhalten, Bienenverlusten, Krankheiten, Verhaltensauffälligkeiten, usw.
Es sind keine Verfahren zur Überwachung von Nutztieren bekannt, die eine effektive (halb)automatisierte Überwachung von Bienenvölkern ermöglichen. Daher müssen Imker, die mit ihren Bienenvölkern wandern, d.h. ihre Bienenvölker dorthin bringen und aufstellen, wo gerade Pflanzen blühen und eine gute Tracht vorzufinden ist, für die Überwachung und Führung der Bienenvölker immer vor Ort sein, was einen erheblichen Aufwand bedeutet.
Der vorliegenden Erfindung liegt somit die Aufgabe zugrunde, ein Konzept zu schaffen, das die oben genannten Nachteile vermindert bzw. vermeidet.
Diese Aufgabe wird gelöst durch die unabhängigen Patentansprüche.
Ausführungsbeispiele der vorliegenden Erfindung schaffen eine Vorrichtung zum Erhalten einer Information über ein oder mehrere Lebewesen. Die Vorrichtung weist einen Aktor, einen Sensor und eine Auswerteeinrichtung auf. Der Aktor ist ausgebildet, um ein Anregungssignal zur Anregung des oder der Lebewesen auszugeben. Der Sensor ist ausgebildet, um ein von dem oder der Lebewesen als Reaktion auf das Anregungssignal erzeugtes Signal zu detektieren, um ein detektiertes Signal zu erhalten. Die Auswerteeinrichtung ist ausgebildet, um das detektierte Signal auszuwerten, um die Information über das oder die Lebewesen zu erhalten.
Der vorliegenden Erfindung liegt die Idee zugrunde, mittels eines Aktors ein Anregungssignal zur Anregung des oder der Lebewesen (z.B. Insekten) auszugeben und anstelle eines zufälligen und von äußeren Parametern (wie z.B. Temperatur und Luftfeuchtigkeit) abhängigen Signals, ein von dem oder den Lebewesen als Reaktion auf das Anregungssignal erzeugtes Signal zu detektieren und auszuwerten, um die Information über das oder die Lebewesen zu erhalten.
Weitere Ausführungsbeispiele schaffen ein System mit der oben beschriebenen Vorrichtung zum Erhalten einer Information über ein oder mehrere Lebewesen und einer Einrichtung zum Aufnehmen eines Gutes. Die Vorrichtung ist dabei derart an oder in der Einrichtung angeordnet, dass das durch die Einrichtung aufgenommene Gut durch die Vorrichtung überwachbar ist.
Weitere Ausführungsbeispiele schaffen ein System mit der oben beschriebenen Vorrichtung zum Erhalten einer Information über ein oder mehrere Lebewesen und einer Einrichtung zum Aufnehmen eines oder mehrerer Lebewesen. Die Vorrichtung ist dabei derart an oder in der Einrichtung angeordnet, dass ein oder mehrere durch die Einrichtung aufgenommenen Lebewesen mit der Vorrichtung überwachbar sind.
Weitere Ausführungsbeispiele schaffen ein Verfahren zum Erhalten einer Information über ein oder mehrere Lebewesen. Das Verfahren umfasst das Ausgeben eines Anregungssignals zur Anregung des oder der Lebewesen. Ferner umfasst das Verfahren das Detektieren eines von dem oder den Lebewesen als Reaktion auf das Anregungssignal erzeugten Signals, um ein detektiertes Signal zu erhalten. Ferner umfasst das Verfahren das Auswerten des detektierten Signals, um die Information über das oder die Lebewesen zu erhalten.

Weitere Ausführungsbeispiele schaffen eine Vorrichtung zum Erhalten einer Information über einen Schädling oder mehrere Schädlinge in Schüttgut. Die Vorrichtung umfasst einen Aktor zum Ausgeben eines Anregungssignals zur Anregung des Schüttguts, des Schädlings oder der Schädlinge, einen Sensor zum Detektieren eines ansprechend auf die Anregung erzeugten Signals, um ein detektiertes Signal zu erhalten, und eine Auswerteeinrichtung zum Auswerten des detektierten Signals, um die Information über den Schädling oder die Schädlinge in dem Schüttgut zu erhalten.
Ausführungsbeispiele der vorliegenden Erfindung werden bezugnehmend auf die beiliegenden Figuren näher erläutert. Es zeigen:
- Fig. 1: ein schematisches Blockschaltbild einer Vorrichtung zum Erhalten einer Information über ein oder mehrere Lebewesen, gemäß einem Ausführungsbeispiel der vorliegenden Erfindung;
- Fig. 2: ein schematisches Blockschaltbild einer Vorrichtung zum Erhalten einer Information über ein oder mehrere Lebewesen, gemäß einem Ausführungsbeispiel der vorliegenden Erfindung;
- Fig. 3: eine schematische Ansicht eines Systems 90 gemäß einem Ausführungsbeispiel der vorliegenden Erfindung;
- Fig. 4: ein Flussdiagramm eines Verfahrens zum Erhalten einer Information über ein oder mehrere Lebewesen, gemäß einem Ausführungsbeispiel der vorliegenden Erfindung;
- Fig. 5: ein Flussdiagramm eines Verfahrens zur akustischen Mustererkennung;
- Fig. 6a: in einem Diagramm eine Amplitude des detektierten Signals aufgetragen über die Zeit am Beispiel einer Larve der Art Sitophilus granarius (Kornkäfer);
- Fig. 6b: ein Spektrogramm des in Fig. 6a gezeigten detektierten Signals;
- Fig. 6c: in einem Diagramm eine Amplitude des detektierten Signal aufgetragen über die Zeit am Beispiel eines Käfers der Art Sitophilus granarius (Kornkäfer);
- Fig. 6d: ein Spektrogramm des in Fig. 6c gezeigten detektierten Signals.

In der nachfolgenden Beschreibung der Ausführungsbeispiele der Erfindung werden in den Figuren gleiche oder gleichwirkende Elemente mit dem gleichen Bezugszeichen versehen, so dass deren Beschreibung in den unterschiedlichen Ausführungsbeispielen untereinander austauschbar ist.

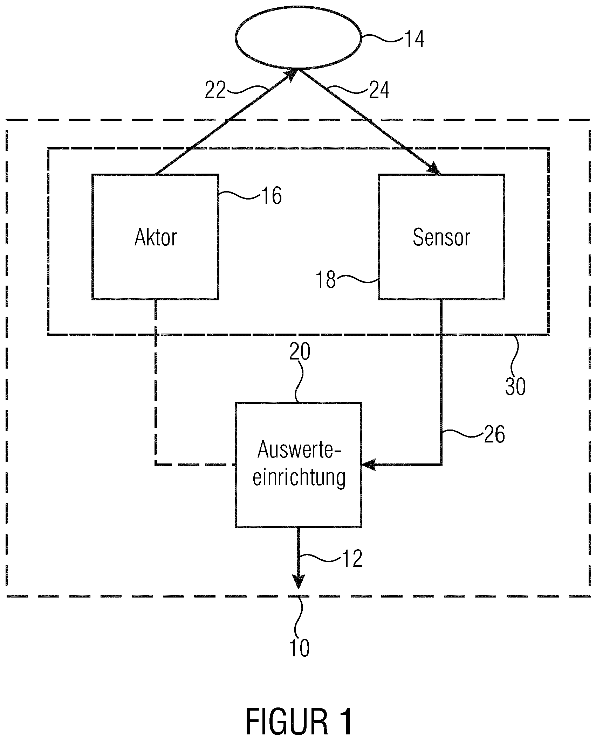

Fig. 1 zeigt ein schematisches Blockschaltbild einer Vorrichtung 10 zum Erhalten einer Information 12 über ein oder mehrere Lebewesen 14. Die Vorrichtung 10 weist einen Aktor 16, einen Sensor 18 und eine Auswerteeinrichtung 20 auf. Der Aktor 16 ist ausgebildet, um ein Anregungssignal 22 zur Anregung des oder der Lebewesen 14 auszugeben. Der Sensor 18 ist ausgebildet, um ein von dem oder der Lebewesen 14 als Reaktion auf das Anregungssignal 22 erzeugtes Signal 24 zu detektieren, um ein detektiertes Signal 26 zu erhalten. Die Auswerteeinrichtung 20 ist ausgebildet, um das detektierte Signal 26 auszuwerten, um die Information 12 über das oder die Lebewesen 14 zu erhalten.

Der vorliegenden Erfindung liegt die Idee zugrunde, mittels eines Aktors 16 ein Anregungssignal 22 zur Anregung des oder der Lebewesen 14 auszugeben und anstelle eines zufälligen und von äußeren Parametern (wie z.B. Temperatur und Luftfeuchtigkeit) abhängendes Signal, ein von dem oder der Lebewesen 14 als Reaktion auf das Anregungssignal 22 erzeugtes Signal 24 zu detektieren und auszuwerten, um die Information 12 über das oder die Lebewesen 14 zu erhalten.

Bei Ausführungsbeispielen kann die Vorrichtung 10 beispielsweise ausgebildet sein, um ein Gut zu überwachen, wobei die Information 12 über das oder die Lebewesen 14 angeben kann, ob das mit der Vorrichtung 10 überwachte Gut von dem oder den Lebewesen 14 befallen ist oder nicht.

Das mit der Vorrichtung 10 überwachte Gut kann beispielsweise ein Nahrungsmittel (z.B. ein Getreide), ein Futtermittel, ein Gebäude, ein Bauwerk, ein Naturholz, eine Holzkonstruktion (z.B. ein Holzpolter, Schnittholz oder Holzbauwerk) ein Feld oder Waldstück sein, während das Lebewesen 14 beispielsweise ein Schädling sein kann.

Bei Ausführungsbeispielen kann die Vorrichtung 10 beispielsweise ausgebildet sein, um das oder die Lebewesen 14 zu überwachen, wobei die Information 12 über das oder die Lebewesen 14 einen Zustand des oder der Lebewesen 14 angibt.

Beispielsweise können die zu überwachenden Lebewesen 14 Nutztiere, wie z.B. Bienen, sein, während der Zustand über die Lebewesen 14 beispielsweise eine Verhaltensauffälligkeit, ein Verlust oder eine Krankheit sein kann.

Bei Ausführungsbeispielen kann der Aktor 16 ausgebildet sein, um als Anregungssignal 22 ein akustisches Anregungssignal auszugeben. Das Anregungssignal 22 kann eine Frequenz oder mehrere Frequenzen aufweisen. Die Frequenz bzw. die Frequenzen können fest oder veränderlich (z.B. ansteigend, abfallend oder gezirpt (engl. chirp)) sein. Die Frequenz bzw. die Frequenzen können im Infraschallbereich (unterhalb von ca. 16 Hz), im hörbaren Frequenzbereich (zwischen ca. 16 Hz und 20 kHz) oder im Ultraschallfrequenzbereich (oberhalb von ca. 16 kHz) liegen. Ferner kann das Anregungssignal 22 einen Frequenzbereich aufweisen, der im Infraschallbereich, im hörbaren Frequenzbereich oder im Ultraschallbereich liegt. Des Weiteren kann das Anregungssignal 22 bandbegrenzt sein, wobei eine obere Grenzfrequenz des Anregungssignals 22 nicht größer als 16 Hz, 16 kHz, 20 kHz, 100 kHz, 500 kHz oder 1 GHz ist.

Das von dem oder den Lebewesen 14 in Reaktion auf das Anregungssignal 22 erzeugte Signal 24 kann ebenfalls ein akustisches Signal sein, welches ebenfalls eine Frequenz oder mehrere Frequenzen aufweisen kann, die im Infraschallbereich, im hörbaren Frequenzbereich und/oder im Ultraschallbereich liegen können.

Dementsprechend kann der Sensor 18 ausgebildet sein, um ein Signal 24 im Infraschallbereich, im hörbaren Frequenzbereich und/oder im Ultraschallfrequenzbereich zu detektieren.

Bei Ausführungsbeispielen können der Aktor 16 und der Sensor 18 in einer gemeinsamen Einrichtung, Vorrichtung, oder Einheit 30 implementiert werden, wie dies in Fig. 1 angedeutet ist. Mit anderen Worten, die Vorrichtung 10 kann eine Aktor/Sensor-Kombination 30 aufweisen, die den Aktor 16 und den Sensor 18 umfasst.

Beispielsweise kann die in Fig. 1 gezeigte Aktor/Sensor-Kombination 30 zur akustischen Anregung 22 des oder der Lebewesen 14 (z.B. einer Tierpopulation) im niederfrequenten und/oder hochfrequenten (Ultraschall) Wellenlängenbereich 22 und zum Empfang akustischer Signale 24, die durch das oder die Lebewesen (z.B. die Tierpopulation) 14 emittiert werden, ausgelegt sein.

Bei Ausführungsbeispielen kann die Auswerteeinrichtung 20 ausgebildet sein, um das detektierte Signal 26 zu klassifizieren, um die Information 12 über das oder die Lebewesen 14 zu erhalten. Die Auswerteeinrichtung 20 kann hierzu z.B. einen Speicher aufweisen, in dem Referenzsignale (z.B. typische Schallmuster) abgelegt sind oder ablegbar sind, wobei die Auswerteeinrichtung 20 ausgebildet sein kann, um das detektierte Signal 26 unter Verwendung der Referenzsignale zu klassifizieren. Beispielsweise kann in dem Speicher für jede Klasse zumindest ein Referenzsignal abgelegt sein, wobei die Auswerteeinrichtung 20 ausgebildet sein kann, um das detektierte Signal 26 mit den Referenzsignalen zu vergleichen, um das detektierte Signal zu klassifizieren.

Des Weiteren kann die Auswerteeinrichtung 20 ein System zur Klassifizierung von Schallereignissen umfassen, wobei die Aktor/Sensor-Kombination 30 mit dem System zur Klassifizierung der Schallereignisse verbunden sein kann. Ferner kann die Auswerteeinrichtung 20 eine Datenbank, die typische Schallmuster enthält, aufweisen, wobei die Aktor/Sensor-Kombination 30 und das System zur Klassifizierung der Schallereignisse mit der Datenbank verbunden sein können.

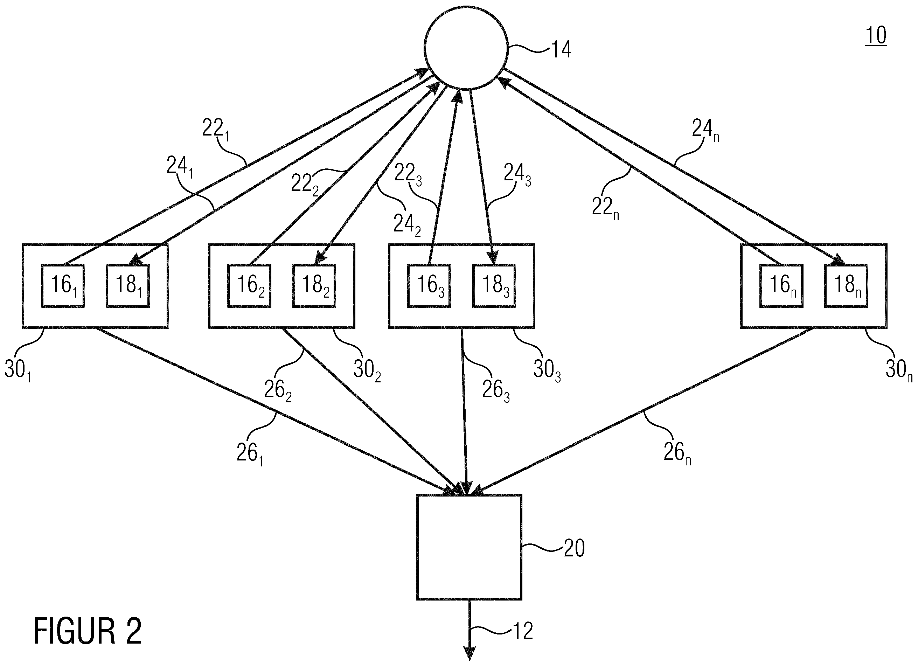

Fig. 2 zeigt ein schematisches Blockschaltbild einer Vorrichtung 10 zum Erhalten einer Information 12 über ein oder mehrere Lebewesen 14. Wie in Fig. 2 zu erkennen ist kann die Vorrichtung n Aktoren 16₁ bis 16ₙ und n Sensoren 18₁ bis 18ₙ aufweisen, wobei n eine natürliche Zahl größer gleich eins ist, n ≥ 1. Die Aktoren 16₁ bis 16ₙ können ausgebildet sein, um Anregungssignale 22₁ bis 22ₙ zur Anregung des oder der Lebewesen 14 auszugeben. Die Sensoren 18₁ bis 18ₙ können ausgebildet sein, um ein von dem oder der Lebewesen 14 als Reaktion auf zumindest eines der Anregungssignale 22₁ bis 22ₙ erzeugtes Signal 24₁ bis 24ₙ zu detektieren, um detektierte Signale 26₁ bis 26ₙ zu erhalten. Die Auswerteeinrichtung 20 kann ausgebildet sein, um die detektierten Signale 26₁ bis 26ₙ auszuwerten, um die Information 12 über das oder die Lebewesen 14 zu erhalten.

Wie in Fig. 2 zu erkennen ist können die Aktoren 16₁ bis 16ₙ und/oder Sensoren 18₁ bis 18ₙ in einem Array angeordnet sein. Die Vorrichtung 10 kann dabei ausgebildet sein, um eine schwenkbare Anregungssignalkeule durch die von den Aktoren 16₁ bis 16ₙ ausgegebenen Anregungssignale 22₁ bis 22ₙ zu erzeugen.

Ferner können jeweils ein Aktor der Aktoren 16₁ bis 16ₙ und ein Sensor der Sensoren 18₁ bis 18ₙ in einer gemeinsamen Einrichtung, Vorrichtung, oder Einheit 30₁ bis 30ₙ implementiert sein. Mit anderen Worten, die Vorrichtung 10 kann n Aktor/Sensor-Kombinationen 30₁ bis 30ₙ aufweisen, wobei jede Aktor/Sensor-Kombination der Aktor/Sensor-Kombinationen 30₁ bis 30ₙ jeweils einen Aktor der Aktoren 16₁ bis 16ₙ und einen Sensor der Sensoren 18₁ bis 18ₙ umfasst bzw. aufweist.

Bei Ausführungsbeispielen kann zumindest eine Aktor/Sensor-Kombination 30 (siehe Fig. 1) oder ein Array von Aktor/Sensor-Kombinationen 30₁ bis 30ₙ (siehe Fig. 2) entsprechend den räumlichen Bedingungen und/oder der vorliegenden Schallausbreitungsgeschwindigkeit angeordnet werden.

Ferner kann mindestens eine Aktor/Sensor-Kombination 30 (siehe Fig. 1) oder ein Array von Aktor/Sensor-Kombinationen 30₁ bis 30ₙ (siehe Fig. 2) an einen Innen- und/oder Außenwand eines Gehäuses (z.B. eines Behälters, eines Silos, eines Schiffsladeraums, eines Containers oder einer Tierbehausung) angeordnet werden.

Des Weiteren kann mindestens eine Aktor/Sensor-Kombination 30 (siehe Fig. 1) oder ein Array von Aktor/Sensor-Kombinationen 30₁ bis 30ₙ (siehe Fig. 2) in einem Innenbereichen eines Gehäuses (z.B. eines Behälters, eines Silos, eines Schiffsladeraums, eines Containers oder einer Tierbehausung) an schallverstärkenden Mitteln wie Blechen, Zwischenwänden oder Schalltrichtern angeordnet werden.

Bei Ausführungsbeispielen kann die Vorrichtung 10 ausgebildet sein, um durch eine Mehrfachanordnung von Aktor/Sensor-Kombinationen 30₁ bis 30ₙ Schallkeulen beim Senden zu schwenken und Verstärkungseffekte durch Wellenüberlagerung zu nutzen (Phased-Array-Technologie, dt. Phasengesteuertes-Feld-Technologie).

Ferner kann die Vorrichtung 10 ausgebildet sein, um durch eine Mehrfachanordnung von Aktor/Sensor-Kombinationen 30₁ bis 30ₙ Schallgeschwindigkeiten im gelagerten Gut (z.B. Material oder Rohstoff) oder in den zu überwachenden Tierpopulation zu bestimmen und die Vorrichtung 10 entsprechend zu kalibrieren.

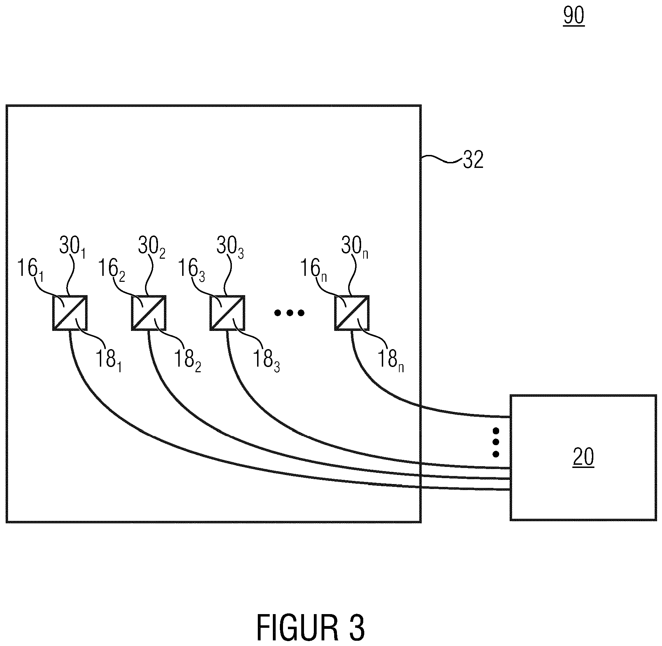

Fig. 3 zeigt eine schematische Ansicht eines Systems 90 gemäß einem Ausführungsbeispiel der vorliegenden Erfindung.

Das System 90 umfasst die oben beschriebene Vorrichtung 10 und eine Einrichtung 92 zum Aufnehmen eines Gutes, oder eine Einrichtung 92 zum Aufnehmen eines oder mehrerer Lebewesen.

Die Vorrichtung 10 umfasst dabei zumindest einen Aktor 16₁ bis 16ₙ und zumindest einen Sensor 18₁ bis 18ₙ. Natürlich kann die Vorrichtung auch zumindest eine Aktor/Sensor-Kombination 30₁ bis 30ₙ umfassen.

Wie in Fig. 3 gezeigt ist können der zumindest eine Aktor 16₁ bis 16ₙ und der zumindest eine Sensor 18₁ bis 18ₙ (oder die zumindest eine Aktor/Sensor-Kombination 30₁ bis 30ₙ) derart an oder in der Einrichtung 90 angeordnet (z.B. verbunden sein), dass das durch die Einrichtung 90 aufgenommene Gut bzw. das oder die durch die Einrichtung 90 aufgenommenen Lebewesen 14 mit der Vorrichtung überwachbar sind.

Ferner können die Aktoren 16 und/oder Sensoren 18 (oder Aktor/Sensor-Kombinationen 30) in Mitteln wie z.B. Kugeln, Zylinder oder ähnlich (autarke Systeme) angeordnet werden, die zunächst nicht mit der Umgebung verbunden sind und zur Durchführung des erfindungsgemäßen Verfahrens an Wandungen aufliegen oder z.B. im Schüttgutstrom mit dem Produkt mitgeführt werden.

Die Aktoren 16 und/oder Sensoren 18 (oder Aktor/Sensor-Kombinationen 30) können dabei mit einer externen Auswerteinrichtung 10 verbunden sein.
Beispielsweise können die Aktoren 16 und/oder Sensoren 18 (oder Aktor/Sensor-Kombinationen 30) können dabei mit der Empfangs- und Auswerteeinheit 20 durch elektrische Leiter (Kabelverbindung), durch optisch Leiter (optische Kopplung) oder durch elektromagnetische Strahlung (Funkverbindung) verbunden sein. Die Energieversorgung kann dabei ebenfalls über diese Verbindung erfolgen. Natürlich können die Aktoren 16 und/oder Sensoren 18 (oder Aktor/Sensor-Kombinationen 30) auch mittels einer Batterie, einem Akkumulator und/oder einer Methode des Energie-Ernten (engl. Energy-Harvesting) mit Energie versorgt werden.
Ferner können die Aktoren 16 und/oder Sensoren 18 (oder Aktor/Sensor-Kombinationen 30) mit einem System zur Feuchtemessung, Temperaturmessung und/oder optischen Messung zur Qualitätsbestimmung kombiniert werden.
Die Einrichtung 90 kann beispielsweise ein Behälter, ein Silo, ein Schiffsladeraum, ein Container oder eine Tierbehausung sein.

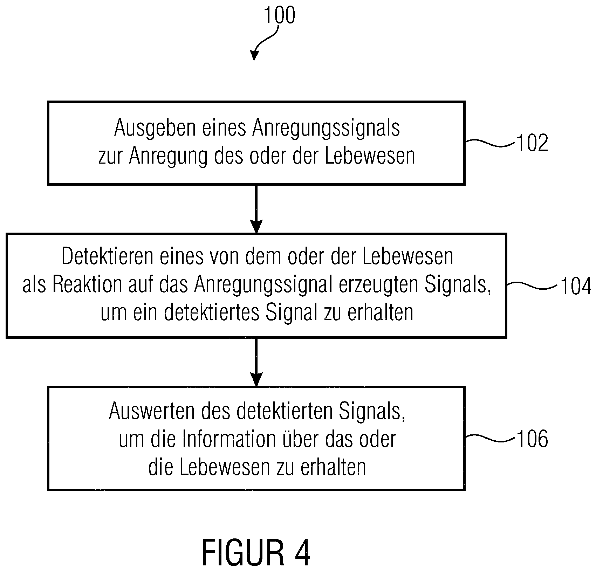

Fig. 4 zeigt ein Flussdiagramm eines Verfahrens 100 zum Erhalten einer Information 14 über ein oder mehrere Lebewesen 14. Das Verfahren 100 umfasst das Ausgeben 102 eines Anregungssignals 22 zur Anregung des oder der Lebewesen 14. Ferner umfasst das Verfahren 100 das Detektieren 104 eines von dem oder den Lebewesen 14 als Reaktion auf das Anregungssignal 22 erzeugten Signals 24, um ein detektiertes Signal 26 zu erhalten. Ferner umfasst das Verfahren 100 das Auswerten 106 des detektierten Signals 26, um die Information 12 über das oder die Lebewesen 14 zu erhalten.
Bei Ausführungsbeispielen umfasst das Verfahren 100 eine Kombination aus akustischer Anregung des oder der Lebewesen 14, z.B. einer Tierpopulation, im niederfrequenten und/oder hochfrequenten (Ultraschall) Wellenlängenbereich (z.B. durch den in Fig. 1 gezeigten Aktor 16) und dem Empfang akustischer Signale 24, die durch das oder die Lebewesen 14 (z.B. Tierpopulation) emittiert werden im Bereich niederfrequenter und/oder hochfrequenter Wellen (z.B. durch den in Fig. 1 gezeigten Sensor 18) in Kombination mit Verfahren zur Ortung sowie zur Klassifizierung dieser Schallereignisse.

Die Anregung des oder der Lebewesen (z.B. Tierpopulation) 14 kann in Abhängigkeit von den Umgebungsbedingungen und der vorliegenden Schallausbreitung sowie in Abhängigkeit von der Art des oder der Lebewesen (z.B. Tierart oder Schädlingsart) im festen oder durchgestimmten Frequenzbereich erfolgen.
Das Verfahren 100 basiert auf dem Senden/Empfangen mit mindestens einem kombinierten Aktor/Sensor 30 (siehe Fig. 1), vorzugsweise mit einem Array (Mehrfachanordnung) von kombinierten Aktoren/Sensoren 30₁ bis 30ₙ (siehe Fig. 2), die entsprechend den räumlichen Bedingungen angeordnet sind.
Das Verfahren einer Mehrfachanordnung von Aktoren/Sensoren 30₁ bis 30ₙ ermöglicht es, Schallkeulen beim Senden zu schwenken und Verstärkungseffekte durch Wellenüberlagerung zu nutzen (Phased-Array-Technologie).
Das Verfahren der Mehrfachanordnung von Aktoren/Sensoren 30₁ bis 30ₙ ermöglicht es weiterhin, Schallgeschwindigkeiten im gelagerten Gut (z.B. Material oder Rohstoff) bzw. in der Tierpopulation zu bestimmen und das Verfahren entsprechend anzupassen.
Die Art der Anordnung und Ausführung der Aktor/Sensor-Kombination 30₁ bis 30ₙ ermöglicht es, z.B. mit niedrigen Frequenzen das oder die Lebewesen (z.B. Tierpopulation) 14 anzuregen und hohe Frequenzen zu empfangen oder umgekehrt, sowie jedoch auch im ähnlichen bzw. gleichen Frequenzbereich zu arbeiten.
Das oben beschriebene Verfahren 100 (welches z.B. eine Klassifikation der empfangenen Schallwellen aufweisen kann) ermöglicht die Überwachung (Monitoring) und Bekämpfung von Schädlingen 14. Beispielsweise kann ein Vorhandensein eines Schädlingsbefalls 14 bestimmt bzw. ermittelt werden. Ferner kann eine Art der Schädlinge 14 bestimmt werden. Des Weiteren kann ein Umfang oder Ausmaß des Schädlingsbefalls bestimmt werden. Darüber hinaus kann ein Siedlungsort bzw. eine Position der Schädlinge 14 im Raum bestimmt werden. Das Verfahren 100 kann ferner eine Filterung der relevanten Geräusche von Umgebungs- bzw. Störgeräuschen umfassen.
Darüber hinaus ermöglicht das oben beschriebene Verfahren 100 (welches z.B. eine Klassifikation der empfangenen Schallwellen aufweisen kann) eine Überwachung von Nutztieren, z.B. in der Bienenhaltung. Beispielsweise können verschiedene Zustände, wie z.B. Aufregung, Hunger, Vorhandensein schlupfreifer Weiseln, Schwarmverhalten, Parasitenbefall, fremde Tiere (z.B. Nager) erkannt werden. Das Verfahren 100 kann ferner eine Filterung der relevanten Geräusche von Umgebungs- bzw. Störgeräuschen umfassen.

Darüber hinaus ermöglicht das oben beschriebene Verfahren 100 eine Schädlingsüberwachung an Naturholz und Holzkonstruktionen (Holpolter, Schnittholz, Holzbauwerke) sowohl vor, während als auch nach der Verarbeitung, eine Quarantäneüberwachung und eine Schädlingsüberwachung in Wäldern (lebende Bäume, Totholz) z.B. durch Anbringung von Manschetten, die Aktor/Sensor-Kombinationen 30 tragen.

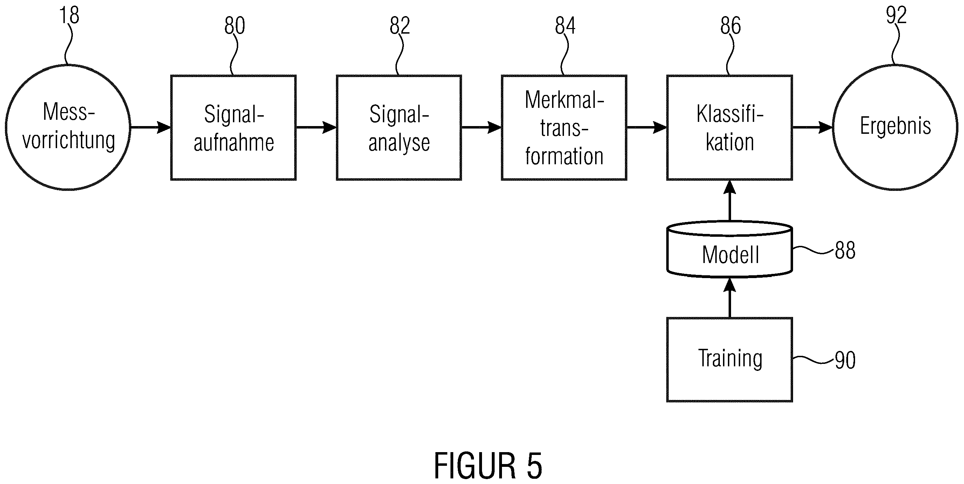

Fig. 5 zeigt ein Flussdiagramm eines Verfahrens zur akustischen Mustererkennung. In einem ersten Schritt 80 kann mittels einer Messvorrichtung (Sensor) 18 eine Signalaufnahme erfolgen. In einem zweiten Schritt 82 kann eine Signalanalyse erfolgen. In einem dritten Schritt 84 kann eine Merkmaltransformation erfolgen. In einem vierten Schritt 86 kann eine Klassifikation, z.B. basierend auf einem Modell 88, welches durch ein Training 90 a gewonnen sein kann, erfolgen, um ein Ergebnis 92 a zu erhalten.
Eine Ausführungsform der akustischen Mustererkennung beinhaltet somit die statistische Folgenklassifikation mit Hidden-Markov-Modellen. Es sind jedoch auch andere Methoden der akustischen Mustererkennung einsetzbar.
Ferner kann das akustische Verfahren mit einem Verfahren der Feuchtemessung und/oder Temperaturmessung, und/oder einem optischen Messverfahren zur Qualitätsbestimmung kombiniert werden.

Weitere Ausführungsbeispiele schaffen eine Vorrichtung 10 zum Erhalten einer Information 12 über einen Schädling oder mehrere Schädlinge 14 in Schüttgut. Die Vorrichtung 10 umfasst einen Aktor 16 zum Ausgeben eines Anregungssignals 22 zur Anregung des Schüttguts, des Schädlings oder der Schädlinge 14, einen Sensor 18 zum Detektieren eines ansprechend auf die Anregung erzeugten Signals 24, um ein detektiertes Signal 26 zu erhalten, und eine Auswerteeinrichtung 20 zum Auswerten des detektierten Signals 26, um die Information 12 über den Schädling oder die Schädlinge 14 in dem Schüttgut zu erhalten.
Dabei kann das erzeugte Signal 24 als Reaktion auf das Anregungssignal 22 von dem Schädling oder der Schädlinge erzeugt werden.
Ferner kann das erzeugte Signal 24 von einer Resonanzfrequenz (oder mehreren Resonanzfrequenzen) des Schüttguts abhängig sein, wobei die Resonanzfrequenz des Schüttguts von einem Befall des Schüttguts mit dem Schädling oder der Schädlinge abhängig ist.

Die Resonanzfrequenz des Schüttguts ist dabei von der Resonanzfrequenz bzw. Eigenfrequenz der einzelnen Elemente (z.B. Körner) des Schüttguts abhängig. Beispielsweise können befallene Körner des Schüttguts eine andere Resonanzfrequenz bzw. Eigenfrequenz aufweisen, als nicht befallene Körner. Die Resonanzfrequenz des Schüttguts kann somit bei Befall mit dem Schädling oder der Schädlinge verschoben werden. Ferner ist es auch möglich, dass das Schüttgut bedingt durch befallene und nicht befallene Körner mehr als eine Resonanzfrequenz aufweist.
Somit ist es möglich entweder den Schädling oder die Schädlinge mit dem Anregungssignal 22 anzuregen und ein als Reaktion auf das Anregungssignal von dem Schädling oder den Schädlingen erzeugtes Signal 24 zu detektieren, oder das Schüttgut anzuregen und ansprechend auf die Anregung des Schüttguts ein von der Resonanzfrequenz (bzw. Eigenfrequenz) des Schüttguts abhängiges Signal zu detektieren. Natürlich können sowohl der Schädling oder die Schädlinge als auch das Schüttgut gleichzeitig angeregt werden, wobei in diesem Fall das Signal welches dem Schädling oder den Schädlingen (teilweise) erzeugt wird zudem von der Resonanzfrequenz (bzw. Eigenfrequenz) des Schüttguts abhängig ist.
Ferner kann der Aktor 16 ausgebildet sein, um eine Frequenz des Anregungssignals 22 auf eine Resonanzfrequenz des Schüttguts abzustimmen.
Des Weiteren kann der Sensor 18 ausgebildet sein, um ein von der Anregung unabhängiges Referenzsignal zu detektieren, um ein detektiertes Referenzsignal zu erhalten, wobei die Auswerteeinrichtung 20 ferner ausgebildet sein kann, um das detektierte Referenzsignal auszuwerten, um die Information 12 über den Schädling oder die Schädlinge 14 in dem Schüttgut zu erhalten.
Beispielsweise kann der Sensor 18 ausgebildet sein, um das Referenzsignal (zeitlich) vor oder nach der Anregung des Schüttguts bzw. des Schädlings oder der Schädlinge zu detektieren. Die Auswerteeinrichtung 20 kann beispielsweise ausgebildet sein, um das detektierte Referenzsignal mit dem detektierten Signal 26 zu vergleichen, um die Information 12 über den Schädling oder die Schädlinge 14 in dem Schüttgut zu erhalten.
Manche Schädlinge erhöhen die Aktivität ansprechend auf das Anregungssignal, so dass das erzeugte Signal 24 verglichen mit dem Referenzsignal zusätzliche Signalanteile aufweist, während andere Schädlinge die Aktivität ansprechend auf das Anregungssignal reduzieren oder sogar einstellen, so dass in dem erzeugten Signal 24 verglichen mit dem Referenzsignal gewissen Signalanteile reduziert sind oder sogar fehlen.

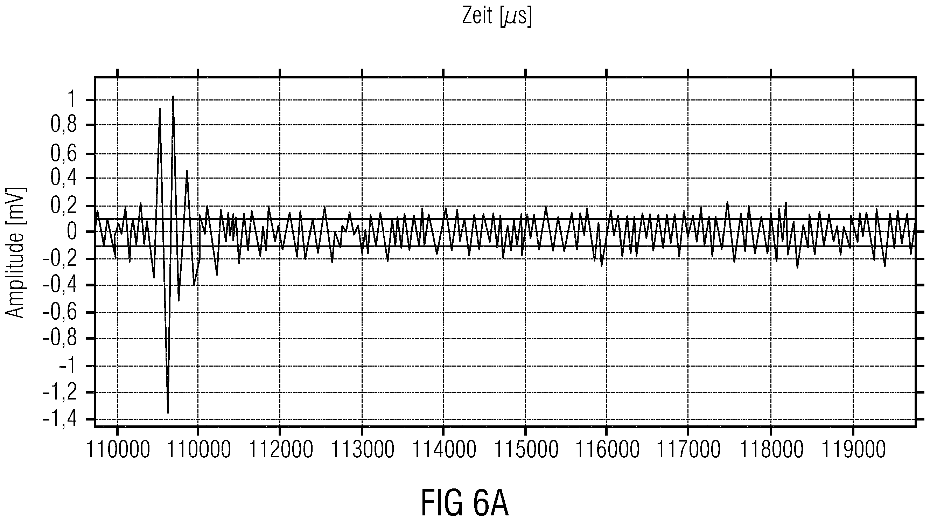

Fig. 6a zeigt in einem Diagramm eine Amplitude des detektierten Signal in mV aufgetragen über die Zeit in s am Beispiel einer Larve der Art Sitophilus granarius (Kornkäfer).

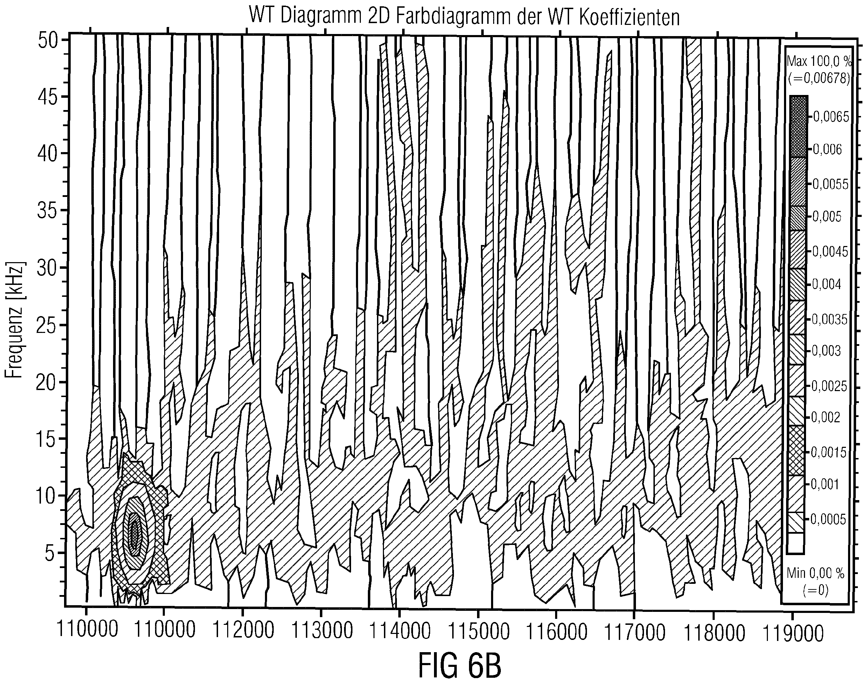

Fig. 6b zeigt ein Spektogramm des in Fig. 6a gezeigten detektierten Signals. Dabei beschreibt in Fig. 6b die ordinate die Frequenz in kHz, während die Abszisse die Zeit in s beschreibt, und der Farb- bzw. Schraffurverlauf die X in % beschreibt.

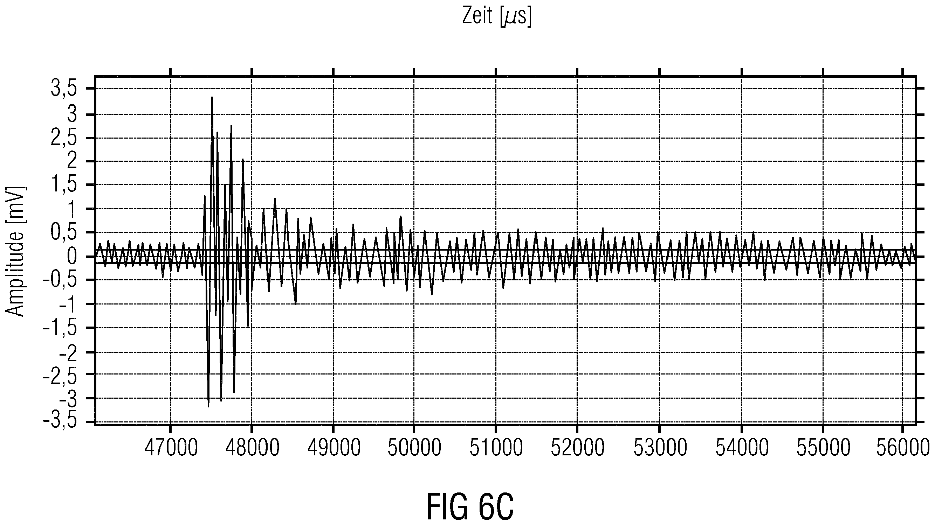

Fig. 6c zeigt in einem Diagramm eine Amplitude des detektierten Signal in mV aufgetragen über die Zeit in s am Beispiel eines Käfers der Art Sitophilus granarius (Kornkäfer).

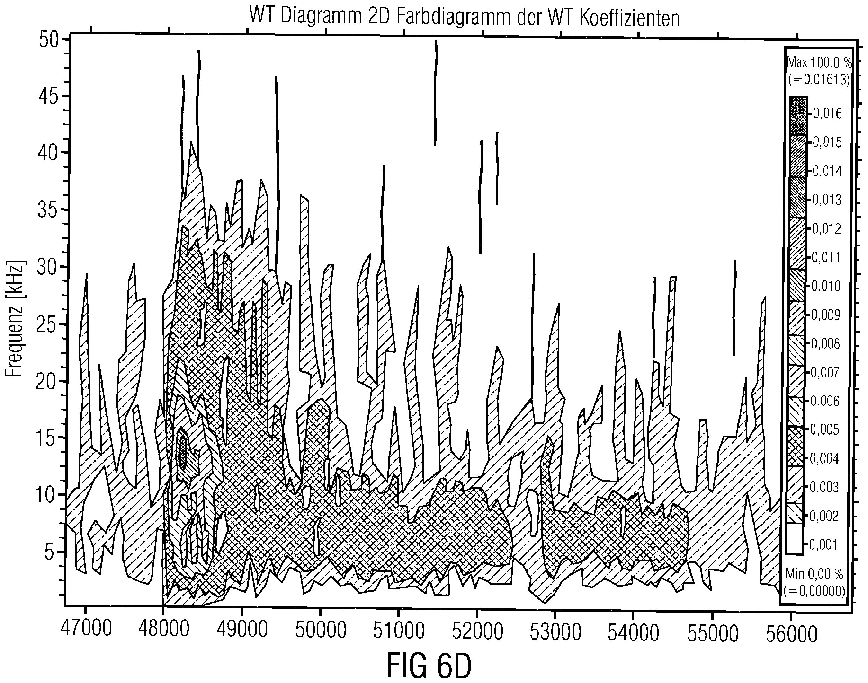

Fig. 6d zeigt ein Spektogramm des in Fig. 6a gezeigten detektierten Signals. Dabei beschreibt in Fig. 6b die Ordinate die Frequenz in kHz, während die Abszisse die Zeit in s beschreibt, und der Farb- bzw. Schraffurverlauf die normierte Signalamplitude in % beschreibt.

Weitere Ausführungsbeispiele schaffen somit eine Vorrichtung zur Detektion von Schädlingen (z.B. Vorratsschädlinge wie z.B. Käfer, Motten, Larven) in allen Entwicklungsstadien in körnigem Schüttgut (z.B. Getreide, Mais, Leguminosen usw.) mit Durchmesser 0,5 mm bis 20 mm. Das Schüttgut besteht aus verschiedenen Körnern des gleichen Typs mit ähnlicher Korngröße.

Beim Senden und Empfangen von Schallsignalen können die Eigenfrequenzen der Körner des Schüttgutes zur Detektion des Schädlingsbefalls und der Korneigenschaften genutzt werden. Körner haben charakteristische Eigenfrequenzen, die ihrer Dimension und ihrem inneren Aufbau entsprechen und auch mit der Kornfeuchte korrelieren.

Das Anregen kann in der charakteristischen Eigenfrequenz der Körner der Schüttung mit den Aktoren erfolgen. Anregungsfrequenz ist im Bereich 1 Hz - 5 MHz, vorzugsweise 10 kHz bis 2 MHz, besonders vorzugsweise 100 kHz bis 1 MHz.

Dabei können Eigenfrequenzunterschiede zwischen vollen, unbefallenen Körnern und hohlen befallenen Körnern erkannt werden.

Der Empfang von Schüttgutschwingungen kann im Frequenzbereich von 10 Hz bis 5 MHz, vorzugsweise 10 kHz bis 2 MHz, besonders vorzugsweise 100khz bis 1 MHz erfolgen.

Die Erkennung eines Schädlingsbefalls kann durch zeitweises Aktivieren bzw. Stoppen der Schädlingsaktivität mit folgenden Schritten ermittelt werden:
- Aussenden von akustischen Wellen im Bereich von 1 Hz bis 20 kHz, vorzugsweise 10 Hz bis 10 kHz, besonders vorzugsweise 10 Hz bis 1 kHz;
- Empfang der Schallgeräusche im Schütthut mit und ohne zusätzlichem Schalleintrag im Frequenzbereich von 1 Hz bis 20 kHz, vorzugsweise 10 Hz bis 20 kHz (Fig. 6a bis 6d);
- Differenzbildung zwischen beiden Zuständen zur Eliminierung der Umgebungsgeräusche;
- Verwendung von bis zu 10 Empfängern gleichzeitig im Bereich des Schüttgut, mindestens aber ein Sender und ein Empfänger;

Ein Schädlingsbefall kann durch einen automatischen Probennehmer oder durch ein Handgerät mittels Eintauchen in die Schüttung detektiert werden. Das Eintauchen erfolgt an verschiedenen Stellen 1 bis 100x, vorzugsweise 3-30x, besonders vorzugsweise 5-10x.

Eine festinstallierte Siloüberwachung kann einen Schädlingsbefall durch Aussenden von Schallsignalen im niederfrequenten Bereich von 10Hz bis 1kHz zum Stoppen oder Aktivieren der Schädlingsaktivität erfolgen und der Empfang der Schädlingsaktivität kann im Frequenzbereich zwischen 1 kHz bis 20 kHz erfolgen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eine Hardware-Apparats), wie zum Beispiel einen Mikroprozessor, einen programmierbaren Computer oder eine elektronische Schaltung. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist. Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Vorrichtung (10) zum Erhalten einer Information (12) über ein oder mehrere Lebewesen (14) einer Tierpopulation, mit folgenden Merkmalen:
einem Aktor (16) zum Ausgeben eines Anregungssignals (22) zur Anregung des oder der Lebewesen (14) der Tierpopulation;
einem Sensor (18) zum Detektieren eines von dem oder den Lebewesen (14) der Tierpopulation als Reaktion auf das Anregungssignal (22) erzeugten Signals (24), um ein detektiertes Signal (26) zu erhalten; und
einer Auswerteeinrichtung (20) zum Auswerten des detektierten Signals (26), um die Information (12) über das oder die Lebewesen (14) der Tierpopulation zu erhalten.

2. Vorrichtung (10) nach Anspruch 1, wobei der Aktor (16) ausgebildet ist, um das Anregungssignal (22) derart auszugeben, dass ein Frequenzbereich des Anregungssignals (22) im Infraschallbereich, im hörbaren Frequenzbereich oder im Ultraschallfrequenzbereich liegt.

3. Vorrichtung (10) nach einem der Ansprüche 1 bis 2, wobei der Sensor (18) ausgebildet ist, um eines von dem oder den Lebewesen (14) der Tierpopulation als Reaktion auf das Anregungssignal (22) erzeugten Signals (24) im Infraschallfrequenzbereich, im hörbaren Frequenzbereich oder im Ultraschallfrequenzbereich zu detektieren.

4. Vorrichtung (10) nach einem der Ansprüche 1 bis 3, wobei die Vorrichtung (10) ausgebildet ist, um eine Ausbreitungsgeschwindigkeit des Anregungssignals (22) zu ermitteln, und um basierend auf der bestimmten Ausbreitungsgeschwindigkeit der Anregungssignals (22) die Information (12) über das oder die Lebewesen (14) der Tierpopulation zu erhalten.

5. Vorrichtung (10) zum Erhalten einer Information (12) über ein Schädling oder mehrere Schädlinge (14) in Schüttgut, mit folgenden Merkmalen:
einem Aktor (16) zum Ausgeben eines Anregungssignals (22) zur Anregung des Schüttguts, des Schädlings oder der Schädlinge (14);
einem Sensor (18) zum Detektieren eines ansprechend auf die Anregung erzeugten Signals (24), um ein detektiertes Signal (26) zu erhalten;
einer Auswerteeinrichtung (20) zum Auswerten des detektierten Signals (26), um die Information (12) über den Schädling oder die Schädlinge (14) in dem Schüttgut zu erhalten.

6. Vorrichtung (10) nach Anspruch 5, wobei der Aktor (16) ausgebildet ist, um eine Frequenz des Anregungssignals (22) auf eine Resonanzfrequenz des Schüttguts abzustimmen.

7. Vorrichtung (10) nach einem der Ansprüche 5 bis 6, wobei der Sensor (18) ferner ausgebildet ist, um ein von der Anregung unabhängiges Referenzsignal zu detektieren, um ein detektiertes Referenzsignal zu erhalten;
wobei die Auswerteeinrichtung (20) ferner ausgebildet ist, um das detektierte Referenzsignal auszuwerten, um die Information (12) über den Schädling oder die Schädlinge (14) in dem Schüttgut zu erhalten.

8. System (90), mit folgenden Merkmalen:
einer Vorrichtung (10) nach einem der Ansprüche 1 bis 7; und
einer Einrichtung (92) zum Aufnehmen eines Gutes, wobei die Vorrichtung (10) an oder in der Einrichtung (92) derart angeordnet ist, dass ein durch die Einrichtung (92) aufgenommenes Gut durch die Vorrichtung (10) überwachbar ist; oder
einer Einrichtung (92) zum Aufnehmen eines oder mehrerer Lebewesen (14), wobei die Vorrichtung (10) an oder in der Einrichtung (92) derart angeordnet ist, dass ein oder mehrere Lebewesen (14) durch die Vorrichtung (10) überwachbar sind.

9. Verfahren (100) zum Erhalten einer Information (12) über ein oder mehrere Lebewesen (14) einer Tierpopulation, wobei das Verfahren aufweist:
Ausgeben (102) eines Anregungssignals (22) zur Anregung des oder der Lebewesen (14) der Tierpopulation;
Detektieren (104) eines von dem oder den Lebewesen (14) der Tierpopulation als Reaktion auf das Anregungssignal (22) erzeugten Signals, um ein detektiertes Signal (26) zu erhalten; und
Auswerten (106) des detektierten Signals (26), um die Information (12) über das oder die Lebewesen (14) der Tierpopulation zu erhalten.

10. Verfahren (100) zum Erhalten einer Information (12) über ein Schädling oder mehrere Schädlinge (14) in Schüttgut, wobei das Verfahren aufweist:
Ausgeben eines Anregungssignals (22) zur Anregung des Schüttguts, des Schädlings oder der Schädlinge (14);
Detektieren eines ansprechend auf die Anregung erzeugten Signals (24), um ein detektiertes Signal (26) zu erhalten; und
Auswerten des detektierten Signals (26), um die Information (12) über den Schädling oder die Schädlinge (14) in dem Schüttgut zu erhalten.

11. Computerprogramm mit einem Programmcode zur Durchführung des Verfahrens (100) nach Anspruch 9 oder 10, wenn das Computerprogramm auf einem Computer abläuft.

12. Verwenden der Vorrichtung nach Anspruch 1, um ein Gut zu überwachen, wobei die Information (12) über das oder die Lebewesen (14) der Tierpopulation angibt, ob das mit der Vorrichtung (10) überwachte Gut von dem oder den Lebewesen (14) der Tierpopulation befallen ist.

13. Verwenden der Vorrichtung nach Anspruch 1, um das oder die Lebewesen (14) der Tierpopulation zu überwachen, wobei die Information (12) über das oder die Lebewesen (14) der Tierpopulation einen Zustand des oder der Lebewesen (14) der Tierpopulation angibt.

## Claims

1. Device (10) for obtaining information (12) about one or more living beings (14) of an animal population, comprising:
an actuator (16) for outputting an excitation signal (22) for exciting the living being(s) (14) of the animal population;
a sensor (18) for detecting a signal (24) generated by the living being(s) (14) of the animal population in reaction to the excitation signal (22), so as to obtain a detected signal (26); and
an evaluator (20) for evaluating the detected signal (26) so as to obtain the information (12) about the living being(s) (14) of the animal population.

2. Device (10) as claimed in claim 1, wherein the actuator (16) is configured to output the excitation signal (22) in such a manner that a frequency range of the excitation signal (22) is within the infrasonic frequency range, within the audible frequency range or within the ultrasonic frequency range.

3. Device (10) as claimed in any of claims 1 to 2, wherein the sensor (18) is configured to detect a signal (24), generated by the living being(s) (14) of the animal population in reaction to the excitation signal (22), within the infrasonic frequency range, within the audible frequency range or within the ultrasonic frequency range.

4. Device (10) as claimed in any of claims 1 to 3, the device (10) being configured to determine a propagation speed of the excitation signal (22) and to obtain the information (12) about the living being(s) (14) of the animal population on the basis of the determined propagation speed of the excitation signal (22).

5. Device (10) for obtaining information (12) about a pest or several pests (14) within a bulk material, comprising:
an actuator (16) for outputting an excitation signal (22) for exciting the bulk material, the pest or the pests (14);
a sensor (18) for detecting a signal (24) generated in response to the excitation, so as to obtain a detected signal (26);
an evaluator (20) for evaluating the detected signal (26) so as to obtain the information (12) about the pest(s) (14) within the bulk material.

6. Device (10) as claimed in claim 5, wherein the actuator (16) is configured to tune a frequency of the excitation signal (22) to a resonant frequency of the bulk material.

7. Device (10) as claimed in any of claims 5 to 6, wherein the sensor is further configured to detect a reference signal that is independent of the excitation, so as to obtain a detected reference signal;
wherein the evaluator (20) is further configured to evaluate the detected reference signal so as to obtain the information (12) about the pest(s) (14) within the bulk material.

8. System (90), comprising:
a device (10) as claimed in any of claims 1 to 7; and
means (92) for accommodating a commodity, the device (10) being arranged at or within the means (92) such that a commodity accommodated by the means (92) can be monitored by means of the device (10); or
means (92) for accommodating one or more living beings (14), the device (10) being arranged at or within the means (92) such that one or more living beings (14) can be monitored by means of the device (10).

9. Method (100) of obtaining information (12) about one or more living beings (14) of an animal population, the method comprising:
outputting (102) an excitation signal (22) for exciting the living being(s) (14) of the animal population;
detecting (104) a signal generated by the living being(s) (14) of the animal population in reaction to the excitation signal (22), so as to obtain a detected signal (26); and
evaluating (106) the detected signal (26) so as to obtain the information (12) about the living being(s) (14) of the animal population.

10. Method (100) of obtaining information (12) about a pest or several pests (14) within a bulk material, the method comprising:
outputting an excitation signal (22) for exciting the bulk material or the pest(s) (14);
detecting a signal (24) generated in response to the excitation, so as to obtain a detected signal (26); and
evaluating the detected signal (26) so as to obtain the information (12) about the pest(s) (14) within the bulk material.

11. Computer program having a program code for performing the method (100) as claimed in claim 9 or 10, when the computer program runs on a computer.

12. Usage of the device as claimed in claim 1, for monitoring a commodity, the information (12) about the living being(s) (14) of an animal population indicating whether the commodity monitored by the device (10) is infested with the living being(s) (14) of the animal population.

13. Usage of the device as claimed in claim 1, for monitoring the living being(s) (14) of an animal population, the information (12) about the living being(s) (14) of an animal population indicating a state of the living being(s) (14) of the animal population.

## Revendications

1. Dispositif (10) pour obtenir une information (12) sur un ou plusieurs organismes vivants (14) d'une population animale, aux caractéristiques suivantes:
un actionneur (16) destiné à sortir un signal d'excitation (22) pour exciter le ou les être vivants (14) de la population animale;
un capteur (18) destiné à détecter un signal (24) généré par le ou les organismes vivants (14) de la population animale en réaction au signal d'excitation (22), pour obtenir un signal détecté (26); et
un dispositif d'évaluation (20) destiné à évaluer le signal détecté (26), pour obtenir l'information (12) sur le ou les organismes vivants (14) de la population animale.

2. Dispositif (10) selon la revendication 1, dans lequel l'actionneur (16) est réalisé pour sortir le signal d'excitation (22) de sorte qu'une plage de fréquences du signal d'excitation (22) se situe dans la plage des infrasons, dans la plage de fréquences audibles ou dans la plage de fréquences ultrasoniques.

3. Dispositif (10) selon l'une des revendications 1 à 2, dans lequel le capteur (18) est réalisé pour détecter un signal (24) généré par le ou les organismes vivants (14) de la population animale en réaction au signal d'excitation (22) dans la plage de fréquences infrasonores, dans la plage de fréquences audibles ou dans la plage de fréquences ultrasoniques.

4. Dispositif (10) selon l'une des revendications 1 à 3, dans lequel le dispositif (10) est réalisé pour déterminer une vitesse de propagation du signal d'excitation (22), et pour obtenir, sur base de la vitesse de propagation déterminée du signal d'excitation (22), l'information (12) sur le ou les organismes vivants (14) de la population animale.

5. Dispositif (10) pour obtenir une information (12) sur un ou plusieurs parasites (14) dans un produit en vrac, aux caractéristiques suivantes:
un actionneur (16) destiné à sortir un signal d'excitation (22) pour exciter le produit en vrac, le ou les parasites (14);
un capteur (18) destiné à détecter un signal (24) généré en réaction à l'excitation, pour obtenir un signal détecté (26);
un dispositif d'évaluation (20) destiné à évaluer le signal détecté (26), pour obtenir l'information (12) sur le ou les parasites (14) dans le produit en vrac.

6. Dispositif (10) selon la revendication 5, dans lequel l'actionneur (16) est réalisé pour accorder une fréquence du signal d'excitation (22) sur une fréquence de résonance du produit en vrac.

7. Dispositif (10) selon l'une des revendications 5 à 6, dans lequel le capteur (18) est par ailleurs réalisé pour détecter un signal de référence indépendant de l'excitation, pour obtenir un signal de référence détecté;
dans lequel le dispositif d'évaluation (20) est par ailleurs réalisé pour évaluer le signal de référence détecté, pour obtenir l'information (12) sur le ou les parasites (14) dans le produit en vrac.

8. Système (90), aux caractéristiques suivantes:
un dispositif (10) selon l'une des revendications 1 à 7; et
un moyen (92) destiné à recevoir un produit, ledit dispositif (10) étant disposé sur ou dans le moyen (92) de sorte qu'un produit reçu par le moyen (92) puisse être surveillé par le dispositif (10); ou
un moyen (92) destiné à recevoir un ou plusieurs organismes vivants (14), le dispositif (10) étant disposé sur ou dans le moyen (92) de sorte qu'un ou plusieurs organismes vivants (14) puissent être surveillés par le dispositif (10).

9. Procédé (100) pour obtenir une information (12) sur un ou plusieurs organismes vivants (14) d'une population animale, le procédé présentant le fait de:
sortir (102) un signal d'excitation (22) pour exciter le ou les organismes vivants (14) de la population animale;
détecter (104) un signal généré par le ou les organismes vivants (14) de la population animale en réaction au signal d'excitation (22), pour obtenir un signal détecté (26); et
évaluer (106) le signal détecté (26), pour obtenir l'information (12) sur le ou les organismes vivants (14) de la population animale.

10. Procédé (100) pour obtenir une information (12) sur un ou plusieurs parasites (14) dans le produit en vrac, le procédé présentant le fait de:
sortir un signal d'excitation (22) pour exciter le produit en vrac, le ou les parasites (14);
détecter un signal (24) généré en réaction à l'excitation, pour obtenir un signal détecté (26); et
évaluer le signal détecté (26), pour obtenir l'information (12) sur le ou les parasites (14) dans le produit en vrac.

11. Programme d'ordinateur avec un code de programme pour mettre en oeuvre le procédé (100) selon la revendication 9 ou 10 lorsque le programme d'ordinateur est exécuté sur un ordinateur.

12. Utilisation du dispositif selon la revendication 1, pour surveiller un produit, dans laquelle l'information (12) sur le ou les organismes vivants (14) de la population animale indique si le produit surveillé par le dispositif (10) est attaqué par le ou les organismes vivants (14) de la population animale.

13. Utilisation du dispositif selon la revendication 1, pour surveiller le ou les organismes vivants (14) de la population animale, dans laquelle l'information (12) sur le ou les organismes vivants (14) de la population animale indique un état du ou des organismes vivants (14) de la population animale.
